(19) Europäisches Patentamt
European Patent Office
Office européen des brevets

(11) Veröffentlichungsnummer: **0 102 510**
**B1**

(12) **EUROPÄISCHE PATENTSCHRIFT**

(45) Veröffentlichungstag der Patentschrift: **07.10.87**

(21) Anmeldenummer: **83107369.7**

(22) Anmeldetag: **27.07.83**

(51) Int. Cl.⁴: **C 07 D 237/14,** C 07 D 237/16, C 07 D 237/18, C 07 D 237/22, C 07 D 237/24

(54) **Substituierte 4-Fluorpyridaz-6-one.**

(30) Priorität: **06.08.82 DE 3229325**

(43) Veröffentlichungstag der Anmeldung: **14.03.84 Patentblatt 84/11**

(45) Bekanntmachung des Hinweises auf die Patenterteilung: **07.10.87 Patentblatt 87/41**

(84) Benannte Vertragsstaaten: **CH DE FR GB IT LI**

(56) Entgegenhaltungen:
**AT - A - 321 926**
**DE - A - 1 948 550**
**DE - B - 1 135 115**
**FR - A - 2 251 330**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Lardon, Hartmut, Dr., Bruesseler Ring 28, D-6700 Ludwigshafen (DE)**
Erfinder: **Seybold, Guenther, Dr., Friedrich-Ebert-Strasse 14, D-6708 Neuhofen (DE)**

**EP 0 102 510 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Verbindungen der allgemeinen Formel I

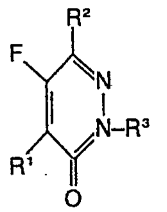

(I)

in der

$R^1$ Fluor, Chlor oder Brom,

$R^2$ Wasserstoff oder Nitro und

$R^3$ durch Chlorocarbonyl, Chlorosulfonyl, Amino, Isocyanato oder Isothiocyanato substituiertes Alkyl, Phenyl oder Naphthyl oder ein ebenso substituierter Heterocyclus sind, wobei das Alkyl noch durch Fluor, Chlor, Brom, $C_1$- bis $C_4$-Alkoxy oder Hydroxycarbonyl und das Phenyl, Naphthyl und der Heterocyclus darüberhinaus durch Hydroxy, Trifluormethyl oder Hydroxysulfonyl weiter substituiert sein können.

Einzelne aliphatische Reste $R^3$ sind z. B. Aminomethyl, 2-Aminoethyl, 1- oder 2-Methyl-2-aminoethyl, 1- oder 2-Hydroxycarbonyl-2-aminoethyl, 3-Aminopropyl, 4-Aminobutyl, 1- oder 2-Fluor-2-aminoethyl, 1- oder 2-Chlor-2-aminoethyl, Chlorocarbonylmethyl, 2-Chlorocarbonylethyl, 1- oder 2-Methyl-2-chlorocarbonylethyl, 2-Chlorosulfonylethyl, oder 1- oder 2-Fluor-2-chlorosulfonylethyl.

Bevorzugte aliphatische Reste $R^3$ sind 2-Chlorocarbonylethylreste, die zusätzlich durch Methyl oder weiteres Chlorocarbonyl substituiert sein können.

Aromatische Reste $R^3$ sind insbesondere Phenyl und Naphthylreste, die wiederum Amino-, Isocyanato- oder Isothiocyanatogruppen oder Carbonsäure- oder Sulfonsäurehalogenidreste tragen und z.B. noch durch Fluor, Chlor, Brom, $C_1$- bis $C_4$-Alkyl oder -Alkoxy, Nitro, Hydroxy, Hydroxycarbonyl oder Hydroxysulfonyl substituiert sein können.

Einzelne aromatische Reste $R^3$ sind z.B.:

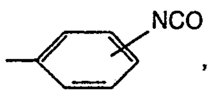 , 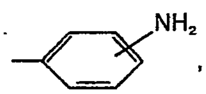 , 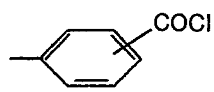 , 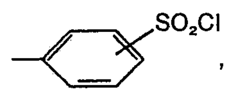 , 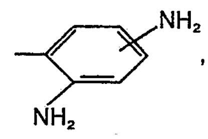 ,

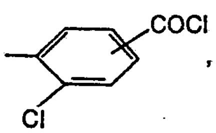 , 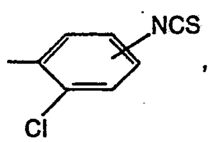 , 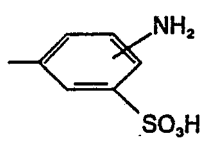 , 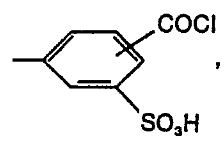 , 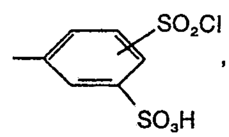 ,

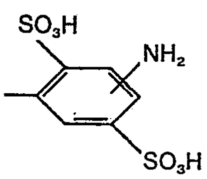 , 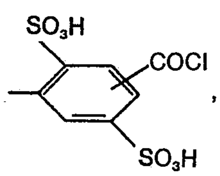 , 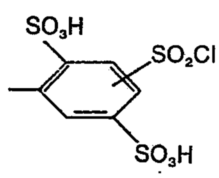 , 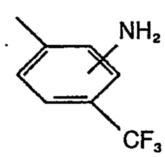 , 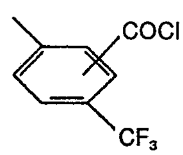 ,

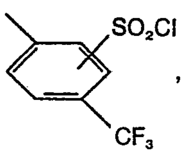 , 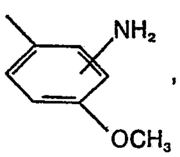 , 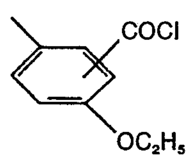 , 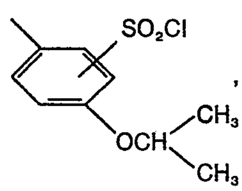 , 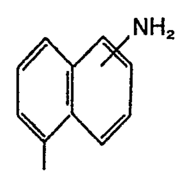 ,

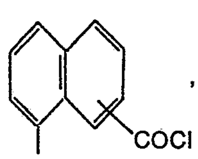 , 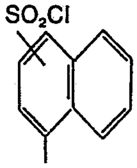 , 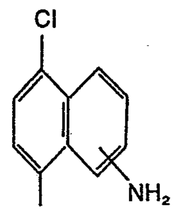 , 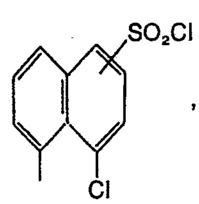 , 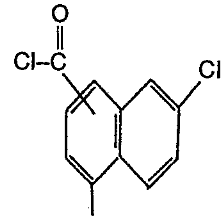 ,

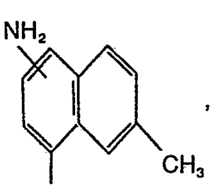 , 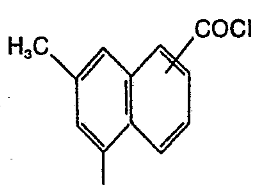 , 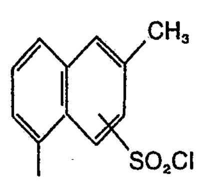 , 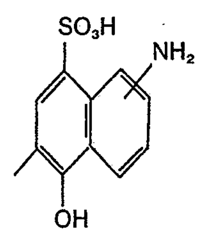 , 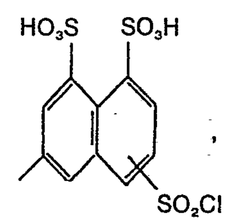 ;

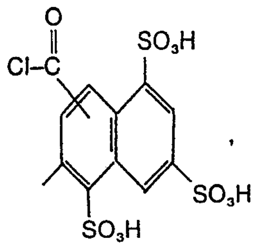 , 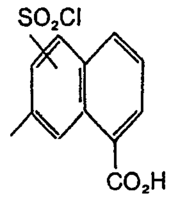 , 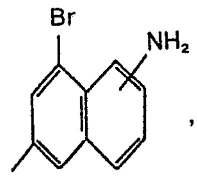 , 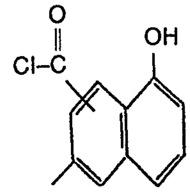 , 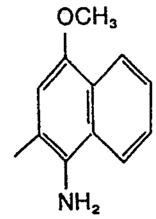

oder 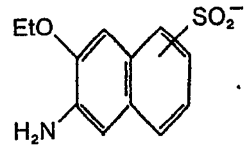 .

Bevorzugte aromatische Reste $R^3$ sind Phenylreste, die Amino-, Chlorocarbonyl-, Chlorosulfonyl-, Isocyanato- oder Isothiocyanatogruppen tragen und zusätzlich durch Methyl, Hydroxysulfonyl oder weiteres Amino substituiert sein können. Heterocyclische Reste $R^3$ sind beispielsweise:

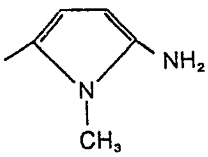 , 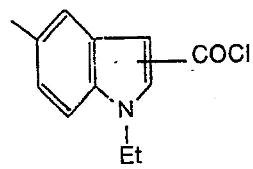 , 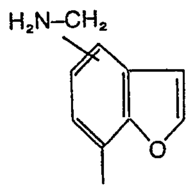 ,

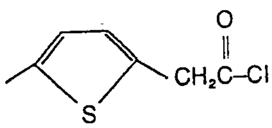 , 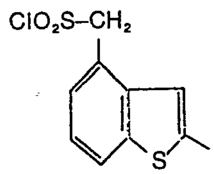 , 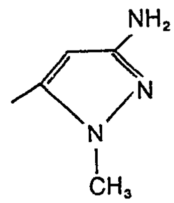 ,

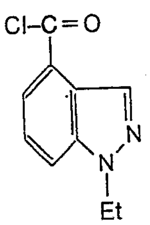 , 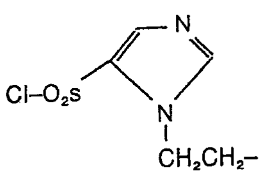 , 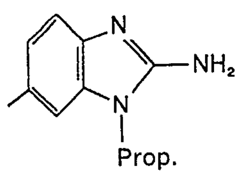 , 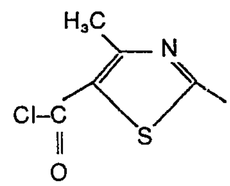 ,

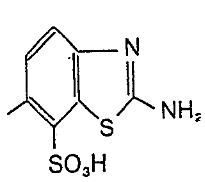 , 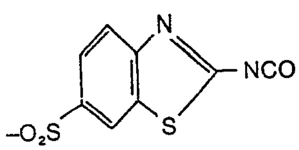 , 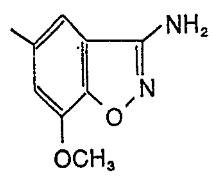 , 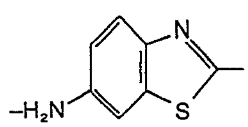 ;

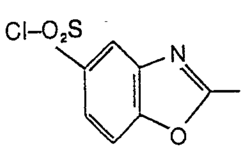 , 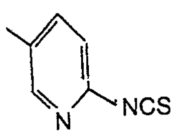 , 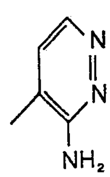 , 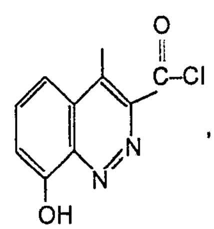 ,

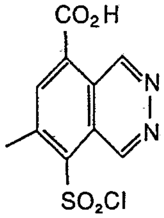, 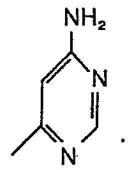, 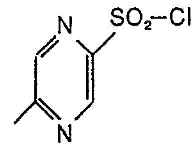, 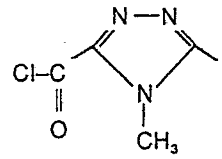,

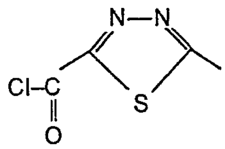, 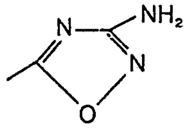 oder 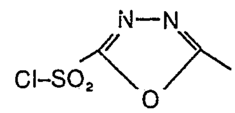

Bevorzugte heterocyclische Reste $R^3$ leiten sich von Thiazol-, Benzthiazol-, Imidazol- oder 1,3,4-Thiadiazolresten ab, die eine Amino-, Chlorocarbonyl-, Chlorosulfonyl-, Isocyanato- oder Isothiocyanatogruppe tragen.

Zur Herstellung der Verbindungen der Formel I kann man Verbindungen der Formel II

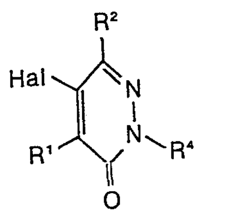 II,

in der Hal Chlor oder Brom ist und $R^1$ und $R^2$ die angegebenen Bedeutungen haben, in die Fluorverbindungen überführen, $R^4$ ist dabei ein Rest $R^3$ oder ein in $R^3$ überführbarer Rest.

Die Überführungen der Verbindungen der Formel II in die Fluorverbindungen erfolgt durch Umsetzung mit Fluorierungsmitteln wie Natriumfluorid, Kaliumfluorid oder Fluorwasserstoff unter nucleophiler Substitution des Halogenatoms in den Verbindungen der Formel II. Bevorzugtes Fluorierungsmittel ist Kaliumfluorid.

Falls $R^4$ nicht mit $R^3$ identisch ist, wird abschliessend noch $R^4$ in $R^3$ umgewandelt, z.B. durch Abspaltung einer Schutzgruppe oder durch Hydrierung oder Reduktion einer Nitrogruppe zur Aminogruppe oder durch die Hydrolyse einer Estergruppe zur Säure und Umwandlung in das entsprechende Säurehalogenid.

Einzelheiten der Herstellung der Verbindungen der Formel I können den Beispielen entnommen werden, in denen sich Angaben über Teile und Prozente, sofern nicht anders vermerkt, auf das Gewicht beziehen.

Verbindungen der Formel II erhält man in an sich bekannter Weise ausgehend von gegebenenfalls substituierten Hydrazinen sowie gegebenenfalls substituierten Mucohalogensäuren, Halogenmaleinsäuren oder Halogenmaleinsäureanhydriden (vgl. z.B. Angew. Chem. 77, 282 (1965); DE-PS 1 420 011; DE-OS 1 420 969; Monatsh. Chem. 99, 15 (1968); Yuki Gosei Kagaku Kyokai Shi 28, 462 (1970); BE-PS 591 996).

Die Verbindungen der Formel I sind wertvolle Anker für Reaktivfarbstoffe, die sich gegenüber den entsprechenden 4-Chlor- oder 4-Brompyridaz-6-onen durch eine sehr viel höhere Reaktivität auszeichnen.

Aus der DE-A-1 948 550 sind fluorierte Pyridazinderivate bekannt, die sich jedoch nicht als Reaktivanker eignen. Gegenüber Farbstoffen, die in der DE-B-1 135 115 beschrieben sind, zeichnen sich Farbstoffe mit erfindungsgemässen Reaktivankern durch eine sehr viel bessere Fixierung schon bei niedrigeren Temperaturen aus.

Beispiel 1:

1-(4′-Aminophenyl)-5-chlor-4-fluorpyridazon-6

a) 4,5-Dichlor-1-(4′-nitrophenyl)-pyridazon-6

In eine Lösung von 96,0 Teilen 4-Nitrophenylhydrazin in einer Mischung aus 1500 Teilen Wasser und 150 Teilen 98%iger Schwefelsäure trägt man 106 Teile Mucochlorsäure ein und lässt das Reaktionsgemisch 3 Stunden lang bei 90 °C rühren. Der gebildete gelbe Feststoff wird abgesaugt, in 1400 Teile 98%ige Schwefelsäure eingetragen und 1,5 Stunden bei 90 °C gerührt. Anschliessend gibt man das erkaltete Reaktionsgemisch auf 3000 Teile Eis, saugt den gebildeten Feststoff ab, wäscht ihn fünfmal me je 500 Teilen Wasser und trocknet ihn im Vakuum.

Ausbeute: 147 Teile (82% d.Th.), Fp.: 228–231 °C
ber. Cl-Gehalt: 24,8%
gef. Cl-Gehalt: 24,3%

b) 5-Chlor-4-fluor-1-(4′-nitrophenyl)-pyridazon-6

143 Teile 4,5-Dichlor-1-(4′-nitrophenyl)-pyridazon-6 und 34,8 Teile Kaliumfluorid werden in 900 Teilen trockenem Toluol suspendiert. Das Toluol destilliert man aus der Reaktionsmischung ab, versetzt letztere mit 750 Teilen trockenem N,N-Dimethylformamid und lässt 30 Minuten lang bei 130 °C rühren. Das erkaltete Gemisch wird in 3500 Teile Eiswasser gegossen, das ausgefallene Produkt abgesaugt, fünfmal mit je 500 Teilen Eiswasser gewaschen, bei Raumtemperatur im Vakuum getrocknet und aus trockenem Toluol umkristallisiert.

Ausbeute: 123 Teile (91% d.Th.), Fp.: 227–230 °C
Analyse:

| | C | H | Cl | F | N | O |
|---|---|---|---|---|---|---|
| ber.: | 44,5 | 1,9 | 13,2 | 7,0 | 15,6 | 17,8 |
| gef.: | 44,8 | 1,8 | 13,0 | 7,1 | 15,5 | – |

$^{19}F$-NMR ($d^7$-DMF) $\delta = +35,5$ ppm (bezogen auf $CF_3CO_2D$)

$^{13}C$-NMR-Spektrum ($CDCl_3$/TMS)

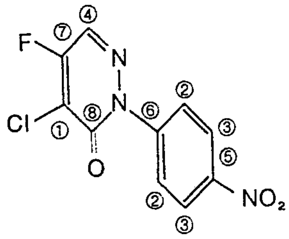

$\delta = 120,8\ C_1$
124,4 $C_2$
126,1 $C_3$
130,9 $C_4$ ($J_{CCF} = 29$ Hz)
145,8 $C_5$
147,6 $C_6$
157,8 $C_7$ ($J_{CF} = 79$ Hz)
160,0 $C_8$

c) 1-(4′-Aminophenyl)-5-chlor-4-fluorpyridazon-6

27,0 Teile 5-Chlor-4-fluor-1-(4′-nitrophenyl)pyridazon-6 werden in 150 Teilen Essigester über 1 Teil Palladiumkatalysator (10% auf Kohle) 3 Tage lang bei 25 °C in 4 bar hydriert. Anschliessend filtriert man das Gemisch aus ausgefallenem Produkt und Katalysator ab, löst das Produkt durch Waschen mit trockenem Methanol aus dem Niederschlag und entfernt aus den vereinigten Filtraten die Lösungsmittel bei Raumtemperatur im Vakuum. Es verbleibt ein Feststoff.

Ausbeute: 23 Teile (96% d.Th.), Fp.: 143–146 °C

Analyse:

| | C | H | Cl | F | N | O |
|---|---|---|---|---|---|---|
| ber.: | 50,1 | 2,9 | 14,8 | 7,9 | 17,6 | 6,7 |
| gef.: | 50,5 | 3,0 | 14,5 | 7,8 | 17,4 | – |

Beispiel 2:

1-(3′-Aminophenyl)-5-chlor-4-fluorpyridazon-6

a) 4,5-Dichlor-1-(3′-nitrophenyl)-pyridazon-6

Zu einer Mischung aus 124 Teilen 3-Nitroanilin, 226 Teilen 38%iger Salzsäure und 500 Teilen Wasser tropft man bei −5 °C eine Lösung von 64,5 Teilen Natriumnitrit in 360 Teilen Wasser, lässt 2 Stunden lang bei 0 °C nachrühren und zerstört überschüssiges Natriumnitrit durch Zugabe von 7 Teilen Amidosulfonsäure. Das Reaktionsgemisch wird vom gebildeten Feststoff abfiltriert und das eisgekühlte Filtrat bei 0 °C zu einer Mischung aus 729 Teilen 39%iger wässriger Natriumhydrogensulfitlösung, 150 Teilen Wasser und 174 Teilen 50%iger Natronlauge getropft. Nach einstündigem Nachrühren bei 0 °C sowie einstündigem Nachrühren bei 20 °C wird auf 40 °C erwärmt, 120 Teile 75%ige Schwefelsäure zugegeben, dann auf 70 °C erwärmt und mit 290 Teilen Kochsalz versetzt. Nach dem Abkühlen auf 0 °C saugt man den entstandenen Niederschlag ab, suspendiert ihn in 1670 Teilen 75%iger Schwefelsäure, trägt bei 55 °C 144 Teile Mucochlorsäure ein, erwärmt auf 90 °C, gibt 920 Teile 96%ige Schwefelsäure zu, lässt 1 Stunde lang bei 90 °C nachrühren und kühlt dann auf 0 °C ab. Das Reaktionsgemisch wird auf Eis gefällt, das Produkt abgesaugt, mit Wasser gewaschen und im Vakuum getrocknet.

Ausbeute: 143 Teile (59% d.Th.), Fp.: 218–221 °C
ber. Cl-Gehalt: 24,8%
gef. Cl-Gehalt: 24,4%

b) 5-Chlor-4-fluor-1-(3′-nitrophenyl)-pridazion-6

143 Teile 4,5-Dichlor-1-(3′-nitrophenyl)-pyridazon-6 werden wie in Beispiel 1b) beschrieben mit 34,8 Teilen Kaliumfluorid eine Stunde lang umgesetzt. Nach Aufarbeitung isoliert man einen Feststoff.

Ausbeute: 132 Teile (98% d.Th.), Fp.: 191–194 °C
ber. Cl-Gehalt: 13,2%, ber. F-Gehalt 7,1%
gef. Cl-Gehalt: 12,9% gef. F-Gehalt 7,3%

c) 1-(3′-Aminophenyl)-5-chlor-4-fluorpyridazon-6

107 Teile 5-Chlor-4-fluor-1-(3′-nitrophenyl)-pyridazon-6 werden in 1000 Teilen eines Gemisches aus 90% n-Butylacetat und 10% n-Butanol über 30 Teilen Raney-Nickel 36 Stunden lang bei 50 °C und 5 bar hydriert. Anschliessend filtriert man das Gemisch aus ausgefallenem Produkt und Katalysator ab, löst das Produkt durch Waschen mit trockenem Methanol aus dem Niederschlag und entfernt aus den vereinigten Filtraten die Lösungsmittel im Vakuum. Es verbleibt ein Feststoff.

Ausbeute: 89 Teile (94% d.Th.), Fp.: 156–159 °C

Analyse:

| | C | H | Cl | F | N | O |
|---|---|---|---|---|---|---|
| ber.: | 50,1 | 2,9 | 14,8 | 7,9 | 17,6 | 6,7 |
| gef.: | 50,7 | 3,1 | 14,4 | 7,7 | 17,5 | – |

Beispiel 3:

5-Chlor-4-fluor-1-(4′-isocyanatophenyl)-pyridazon-6

In 600 Teilen Essigester werden 143,5 Teile Phosgen gelöst und während des Einleitens von weiteren 99 Teilen Phosgen portionsweise 120 Teile 1-(4′-Aminophenyl)-5-chlor-4-fluor-pyridazon-6 zugegeben. Man lässt 15 Stunden lang bei Raumtemperatur nachrühren, heizt dann auf 77 °C und kocht während des Durchleitens eines Stickstoffatomes 8 Stunden lang unter Rückfluss. Nach dem Abkühlen auf Raumtemperatur wird der entstandene Feststoff abgesaugt, mit Essigester gewaschen und im Vakuum getrocknet.

Ausbeute: 124 Teile (93% d.Th.), Fp.: 273–277 °C (Zers.)

IR: $-N=C=O$ bei 2325 $cm^{-1}$

Analyse:

| | C | H | Cl | F | N | O |
|---|---|---|---|---|---|---|
| ber. | 49,7 | 1,9 | 13,4 | 7,2 | 15,8 | 12,0 |
| gef. | 50,1 | 1,8 | 13,6 | 6,9 | 15,7 | – |

Beispiel 4:

5-Chlor-4-fluor-1-(3′-isocyanatophenyl)-pyridazon-6

Wie in Beispiel 3 beschrieben, lassen sich 120 Teile 1-(3′-Aminophenyl)-5-chlor-4-fluorpyridazon-6 mit überschüssigem Phosgen in das entsprechende Isocyanat überführen, das als Feststoff anfällt.

Ausbeute: 100 Teile (75% d.Th.), Fp.: 165–168 °C

IR: $-N=C=O$ bei 2290 $cm^{-1}$

Analyse:

| | C | H | Cl | F | N | O |
|---|---|---|---|---|---|---|
| ber. | 49,7 | 1,9 | 13,4 | 7,2 | 15,8 | 12,0 |
| gef. | 50,2 | 1,7 | 13,7 | 7,0 | 15,8 | – |

Beispiel 5:

5-Chlor-4-fluor-1-(4′-isothiocyanato-phenyl)-pyridazon-6

Zu einer Mischung aus 2000 Teilen Chloroform und 2000 Teilen Wasser gibt man bei 10 °C 115 Teile Thiophosgen und trägt dann bei der gleichen Temperatur portionsweise 120 Teile 1-(4′-Aminophenyl)-5-chlor-4-fluorpyridazon-6 ein. Durch gleichzeitige Zugabe von insgesamt 180 Teilen Calciumcarbonat wird der pH-Wert bei 5–6 gehalten. Nach zweistündigem Nachrühren bei Raumtemperatur filtriert man vom Calciumcarbonat ab, trennt die wässrige Phase des Filtrats ab, wäscht die Chloroformphase zweimal mit Wasser und trocknet sie über Natriumsulfat. Nach dem Abfiltrieren des Trockenmittels wird das Lösungsmittel bis auf ein geringes Restvolumen eingeengt, Petrolether zugesetzt, der entstandene Niederschlag abgesaugt, mit Petrolether gewaschen und getrocknet.

Ausbeute: 117 Teile (83% d.Th.), Fp.: 142–144 °C (aus Schwerbenzin)

IR: $-N=C=S$ bei 2150, 2190 $cm^{-1}$

Analyse:

| | C | H | Cl | F | N | O | S |
|---|---|---|---|---|---|---|---|
| ber. | 46,9 | 1,8 | 12,6 | 6,7 | 14,9 | 5,7 | 11,4 |
| gef. | 47,2 | 1,8 | 12,4 | 6,7 | 15,0 | – | 11,3 |

Beispiel 6:

5-Chlor-4-fluor-1-(3′-isothiocyanatophenyl)-pyridazon-6

Analog zu Beispiel 5 werden 120 Teile 1-(3′-aminophenyl)-5-chlor-4-pyridazon-6 mit 115 Teilen Thiophosgen in Chloroform/Wasser umgesetzt.

Ausbeute: 111 Teile (79% d.Th.), Fp.: 176–178 °C (aus Schwerbenzin)

IR: $-N=C=S$ bei 2100, 2130 $cm^{-1}$

Analyse:

| | C | H | Cl | F | N | O | S |
|---|---|---|---|---|---|---|---|
| ber. | 46,9 | 1,8 | 12,6 | 6,7 | 14,9 | 5,7 | 11,4 |
| gef. | 46,8 | 1,8 | 12,5 | 6,8 | 14,8 | – | 11,5 |

Beispiel 7:

1-(2′-Amino-4′-hydroxisulfonylphenyl)-5-chlor-4-fluorpyridazon-6

a) 5-Chlor-4-fluor-1-(4′-hydroxisulfonyl-phenyl)-pyridazon-6

In 1600 Teile 24%iges Oleum trägt man 480 Teile 5-Chlor-4-fluor-1-phenylpyridazon-6 (Darstellung siehe Beispiel 23a, b) ein, lässt das Gemisch 5 Stunden lang bei 100 °C rühren, gibt es nach dem Abkühlen auf 4000 Teile Eis, und setzt 1300 Teile Kochsalz zu. Das ausgefallene Produkt wird abgesaugt und aus wenig Wasser umkristallisiert.

Ausbeute: 495 Teile (76% d.Th.), Fp.: >260 °C
ber. Cl-Gehalt: 11,7% ber. F-Gehalt: 6,2%
gef. Cl-Gehalt: 11,6% gef. F-Gehalt: 6,1%

b) 5-Chlor-4-fluor-1-(4′-hydroxisulfonyl-2′-nitrophenyl)-pyridazon-6

455 Teile 5-Chlor-4-fluor-1-(4′-hydroxisulfonyl-phenyl)-pyridazon-6 werden in 2374 Teile 96%ige Schwefelsäure eingetragen. Zu dieser Mischung tropft man bei 20 °C 99 Teile 98%ige Salpetersäure und lässt 24 Stunden lang bei Raumtemperatur nachrühren. Anschliessend wird der Reaktionsansatz auf 3300 Teile Eis gegeben, mit 1050 Teilen Kochsalz versetzt, das ausgefallene Produkt abgesaugt, mit eiskalter, gesättigter wässriger Kochsalzlösung sowie wenig Eiswasser gewaschen und getrocknet.

Ausbeute: 360 Teile (69% d.Th.), Fp.: >260 °C
ber. Cl-Gehalt: 10,2% ber. F-Gehalt: 5,4%
gef. Cl-Gehalt: 10,0% gef. F-Gehalt: 5,3%

c) 1-(2′-Amino-4′-hydroxisulfonylphenyl)-5-chlor-4-fluor-pyridazon-6

Eine Lösung von 350 Teilen 5-Chlor-4-fluor-1-(4′-hydroxisulfonyl-2′-nitrophenyl)-pyridazon-6 in 2600 Teilen Wasser tropft man bei 100 °C zu einem Gemisch aus 320 Teilen Eisenpulver, 34 Teilen Essigsäure und 1300 Teilen Wasser. Nach zehnminütigem Nachkochen bei 100 °C wird durch Zugabe von 34%iger wässriger Ammoniaklösung auf pH 7 gestellt, heiss abfiltriert, das Filtrat durch Zugabe von 40%iger Salzsäure auf pH 3 gestellt, der entstandene Niederschlag abgesaugt, mit wenig Eiswasser gewaschen und getrocknet.

Ausbeute: 195 Teile (61% d.Th.), Fp.: 260 C

Analyse:

| | C | H | Cl | F | N | O | S |
|---|---|---|---|---|---|---|---|
| ber. | 37,6 | 2,2 | 11,1 | 6,0 | 13,1 | 20,0 | 10,0 |
| gef. | 37,3 | 2,4 | 11,0 | 5,9 | 13,0 | – | 10,2 |

Beispiel 8:

1-(6′-Amino-2′-hydroxisulfonyl-4′-methylphenyl)-5-chlor-4-fluor-pyridazon-6

a) 4,5-Dichlor-1-(4′-methylphenyl)-pyridazon-6

In eine 60 °C heisse Mischung aus 465 Teilen p-Tolylhydrazin, 7500 Teilen Wasser und 500 Teilen 30%iger Salzsäure trägt man 644 Teile Mucochlorsäure ein, rührt 2 Stunden lang bei 90 °C, lässt auf 40 °C abkühlen, saugt das ausgefallene Produkt ab und wäscht es mit Wasser.

Ausbeute: 855 Teile (88% d.Th.), Fp.: 148–149 °C (aus Toluol)
ber. Cl-Gehalt: 27,8%
gef. Cl-Gehalt: 27,8%

b) 5-Chlor-4-fluor-1-(4′-methylphenyl)-pyridazon-6

510 Teile 4,5-Dichlor-1-(4′-methylphenyl)-pyridazon-6 werden wie in Beispiel 1b) beschrieben, mit 264 Teilen Kaliumfluorid in 650 Teilen trockenem DMF 3 Stunden lang bei 145 °C umgesetzt. Nach wässriger Aufarbeitung isoliert man einen Feststoff.

Ausbeute: 444 Teile (93% d.Th.), Fp.:

ber. Cl-Gehalt: 14,9% ber. F-Gehalt: 8,0%
gef. Cl-Gehalt: 14,8% gef. F-Gehalt: 8,1%

c) 5-Chlor-4-fluor-1-(2′-hydroxisulfonyl-4′-methyl-6′-nitrophenyl-pyridazon-6

405 Teile 5-Chlor-4-fluor-1-(4′-methylphenyl)-pyridazon-6 werden bei 35–40 °C unter Rühren in 1400 Teile 24%igen Oleums eingetragen. Nach zweistündigem Nachrühren bei 50 °C lässt man unter Kühlung bei 0–5 °C 113 Teile 98%iger Salpetersäure langsam zutropfen und dann über Nacht bei Raumtemperatur nachrühren. Anschliessend wird das Reaktionsgemisch auf 375 Teile Kochsalz und 1200 Teile Eis gegeben, das ausgefallene Produkt abgesaugt, mit kalter gesättigter wässriger Kochsalzlösung, dann mit wenig Eiswasser gewaschen und im Vakuum getrocknet.

Ausbeute: 525 Teile (85% d.Th.), Fp: > 260 °C
ber. Cl-Gehalt: 9,8% ber. F-Gehalt: 5,2%
gef. Cl-Gehalt: 10,0% gef. F-Gehalt: 5,1%

d) 1-(6′-Amino-2′-hydroxisulfonyl-4′-methylphenyl)-5-chlor-4-fluorpyridazon-6

523 Teile 5-Chlor-4-fluor-1-(2′-hydroxisulfonyl-4′-methyl-6′-nitrophenyl)-pyridazon-6 werden in 5000 Teilen Wasser durch Zufügen von Natronlauge bis pH 6 gelöst. Diese Lösung und gleichzeitig 75 Teile 40%ige Salzsäure lässt man bei 95–100 °C in ein kochendes Gemisch aus 2500 Teilen Wasser, 520 Teilen Eisenpulver und 52 Teilen Essigsäure laufen. Nach 30-minütigem Nachrühren bei 100 °C wird heiss filtriert, das Filtrat mit 400 Teilen 40%iger Salzsäure versetzt und auf 0 °C gekühlt. Das ausgefällte Produkt saugt man ab, wäscht es mit wenig Eiswasser und trocknet es im Vakuum.

Ausbeute: 398 Teile (83% d.Th.), Fp.: > 260 °C

Analyse:

| | C | H | Cl | F | N | O | S |
|---|---|---|---|---|---|---|---|
| ber. | 39,6 | 2,7 | 10,6 | 5,7 | 12,6 | 19,2 | 9,6 |
| gef. | 40,0 | 2,6 | 10,4 | 5,8 | 12,6 | – | 9,5 |

Beispiel 9:
1-(4′-Aminophenyl)-5-brom-4-fluorpyridazon-6

a) 4,5-Dibrom-1-(4′-nitrophenyl)-pyridazon-6

Wie in Beispiel 1a) beschrieben, werden 76,5 Teile 4-Nitrophenylhydrazin mit 129 Teilen Mucobromsäure umgesetzt. Nach Aufarbeitung isoliert man einen Feststoff.

Ausbeute: 145 Teile (77% d.Th.), Fp.: 238–240 °C
ber. Br-Gehalt: 42,6%
gef. Br-Gehalt: 42,1%

b) 5-Brom-4-fluor-1-(4′-nitrophenyl)-pyridazon-6

Wie in Beispiel 1b) beschrieben, setzt man 220 Teile 4,5-Dibrom-1-(4′-nitrophenyl)-pyridazon-6 bei 140 °C 4 Stunden lang mit 51,2 Teilen Kaliumfluorid um. Nach Aufarbeitung wird ein Feststoff isoliert.

Ausbeute: 182 Teile (99% d.Th.), Fp.: 207–210 °C
ber. Br-Gehalt: 25,4% ber. F-Gehalt: 6,1%
gef. Br-Gehalt: 25,0% gef. F-Gehalt: 6,0%

c) 1-(4′-Aminophenyl)-5-brom-4-fluorpyridazon-6

Wie in Beispiel 1c) beschrieben, werden 31,4 Teile 5-Brom-4-fluor-1-(4′-nitrophenyl)-pyridazon-6 hydriert. Das Produkt fällt als Feststoff an.

Ausbeute: 27,0 Teile (95% d.Th.), Fp.: 261–265 °C

Analyse:

| | C | H | Br | F | N | O |
|---|---|---|---|---|---|---|
| ber. | 42,3 | 2,5 | 28,1 | 6,7 | 14,8 | 5,6 |
| gef. | 42,9 | 2,4 | 27,6 | 6,8 | 14,7 | – |

Beispiel 10:
1-(3′-Aminophenyl)-5-brom-4-fluorpyridazon-6

a) 4,5-Dibrom-1-(3′-nitrophenyl)-pyridazon-6

Wie in Beispiel 2a) beschrieben, werden 124 Teile 3-Nitroanilin über das entsprechende Hydrazin mit 219 Teilen Mucobromsäure umgesetzt. Nach Aufarbeitung isoliert man den Feststoff.

Ausbeute: 195 Teile (61% d.Th.), Fp.: 220–222 °C
ber. Br-Gehalt: 42,6%
gef. Br-Gehalt: 42,0%

b) 5-Brom-4-fluor-1-(3′-nitrophenyl)-pyridazon-6

Wie in Beispiel 1b) beschrieben, setzt man 220 Teile 4,5-Dibrom-1-(3′-nitrophenyl)-pyridazon-6 bei 140 °C 4 Stunden lang mit 51,2 Teilen Kaliumfluorid um. Nach Aufarbeitung wird ein Feststoff isoliert.

Ausbeute: 179 Teile (97% d.Th.), Fp.: 213–216 °C
ber. Br-Gehalt: 25,4% ber. F-Gehalt: 6,1%
gef. Br-Gehalt: 25,1% gef. F-Gehalt: 6,2%

c) 1-(3′-Aminophenyl)-5-brom-4-fluorpyridazon-6

Wie in Beispiel 2c) beschrieben, werden 31,4 Teile 5-Brom-4-fluor-1-(3′-nitrophenyl)-pyridazon-6 hydriert. Das Produkt fällt als Feststoff an.

Ausbeute: 26,8 Teile (94% d.Th.), Fp.: 136–139 °C

Analyse:

| | C | H | Br | F | N | O |
|---|---|---|---|---|---|---|
| ber. | 42,3 | 2,5 | 28,1 | 6,7 | 14,8 | 5,6 |
| gef: | 42,8 | 2,6 | 27,5 | 6,7 | 14,6 | – |

Analog zu den Beispielen 1–10 konnten folgende 4-Fluorpyridaz-6-one synthetisiert werden:

| Bsp.-Nr. | |
|---|---|
| 11 | 5-Chlor-1-(2′ ,4′-diaminophenyl)-4-fluorpydazon-6 |
| 12 | 5-Brom-4-fluor-1-(4′-isocyanatophenyl)-pyridazon-6 |
| 13 | 5-Brom-4-fluor-1-(3′-isocyanatophenyl)-pyridazon-6 |
| 14 | 5-Brom-4-fluor-1-(4′-isothiocyanatophenyl)-pyridazon-6 |
| 15 | 5-Brom-4-fluor-1-(3′-isothiocyanatophenyl)-pyridazon-6 |
| 16 | 1-(2′-Amino-6′-hydroxisulfonyl-4′-methylphenyl)-5-brom-4-fluorpyridazon-6 |

Nach literaturbekannten Sulfierungsmethoden lassen sich aus den in Beispiel Nr. 1, 2, 9 bzw. 10 beschriebenen Pyridazonen folgende Hydroxisulfonylphenylderivate synthetisieren:

| Bsp.-Nr. | |
|---|---|
| 17 | 1-(4′-Amino-3′-hydroxisulfonylphenyl)-5-chlor-4-fluorpyridazon-6 |
| 18 | 1-(5′-Amino-2′-hydroxisulfonylphenyl)-5-chlor-4-fluorpyridazon-6 |
| 19 | 1-(3′-Amino-4′-hydroxisulfonylphenyl)-5-chlor-4-fluorpyridazon-6 |
| 20 | 1-(4′-Amino-3′-hydroxisulfonylphenyl)-5-brom-4-fluorpyridazon-6 |

Beispiel 21:
5-Chlor-1-(4′chlorocarbonylphenyl)-4-fluorpyridazon-6

a) 4,5-Dichlor-1-(4′-ethoxicarbonylphenyl)-pyridazon-6

In eine Lösung von 76 Teilen 4-Hydrazinobenzoesäure in einer Mischung aus 1500 Teilen Wasser und 150 Teilen 40%iger Salzsäure gibt man 85 Teile Mucochlorsäure, lässt das Reaktionsgemisch 2 Stunden lang bei 80 °C rühren, saugt nach dem Abkühlen den entstandenen Feststoff ab, trägt ihn in 1500 Teile 40%ige Salzsäure ein und lässt 12 Stunden lang bei 60 °C rühren. Die erkaltete Reaktionsmischung wird auf Eis gegeben, und der ausgefallene Feststoff abgesaugt, mit Wasser gewaschen, im Vakuum getrocknet und dann portionsweise in 1100 Teile Thionylchlorid eingetragen. Die Temperatur dieses Reaktionsgemisches steigert man langsam bis auf 80 °C. Nach fünfstündigem Nachrühren bei 80 °C wird überschüssiges Thionylchlorid im Vakuum entfernt, der Rückstand mit 350 Teilen Toluol versetzt und dazu bei 50 °C 69 Teile Ethanol zugetropft. Nach sechsstündigem Rühren bei 80 °C und anschliessendem Abkühlen auf 0 °C wird der entstandene Feststoff abgesaugt, mit Toluol gewaschen und im Vakuum getrocknet.

Ausbeute: 111 Teile (71% d.Th.), Fp.: 188–191 °C
ber. Cl-Gehalt: 22,7%
gef. Cl-Gehalt: 22,5%

b) 5-Chlor-1-(4′-ethoxicarbonylphenyl)-4-fluorpyridazon-6

125 Teile 4,5-Dichlor-1-(4′-ethoxicarbonylphenyl)-pyridazon-6 und 28 Teile Kaliumfluorid werden in 800 Teilen trockenem Toluol suspendiert. Das Toluol destilliert man aus der Reaktionsmischung ab, versetzt letztere mit 700 Teilen trockenem N,N-Dimethylformamid und lässt 60 Minuten lang bei 130 °C rühren. Das erkaltete Gemisch wird in 3000 Teile Eiswasser gegossen, das ausgefallene Produkt abgesaugt, fünfmal mit je 500 Teilen Eiswasser gewaschen, bei Raumtemperatur im Vakuum getrocknet und aus trockenem Schwerbenzin umkristallisiert.

Ausbeute: 98 Teile (83% d.Th.), Fp.: 150–152 °C
Analyse:

| | C | H | Cl | F | N | O |
|---|---|---|---|---|---|---|
| ber. | 52,6 | 3,4 | 12,0 | 6,4 | 9,4 | 16,2 |
| gef. | 53,0 | 3,3 | 11,8 | 6,5 | 9,2 | – |

c) 5-Chlor-1-(4′-chlorocarbonylphenyl)-4-fluorpyridazon-6

89 Teile 5-Chlor-1-(4′-ethoxicarbonylphenyl)-4-fluorpyridazon-6 werden in 1000 Teilen 10%iger Salzsäure 6 Stunden lang bei 95 °C gerührt. Nach dem Erkalten saugt man den Feststoff ab, wäscht ihn zweimal mit je 100 Teilen Eiswasser, trocknet ihn bei Raumtemperatur im Vakuum und trägt ihn in 300 Teile Thionylchlorid ein. Das Reaktionsgemisch wird 3 Stunden lang unter Rückfluss gekocht. Anschliessend destilliert man das überschüssige Thionylchlorid ab und kristallisiert den Rückstand aus trockenem Toluol um.

Ausbeute: 73 Teile (85% d.Th.)
Analyse:

| | C | H | Cl | F | N | O |
|---|---|---|---|---|---|---|
| ber. | 46,0 | 1,7 | 24,7 | 6,6 | 9,8 | 11,2 |
| gef. | 46,3 | 1,6 | 24,5 | 6,7 | 9,9 | – |

Beispiel 22:
5-Chlor-1-(3′-chlorocarbonylphenyl)-4-fluorpyridazon-6

a) 4,5-Dichlor-1-(3′-ethioxicarbonylphenyl)-pyridazon-6

Analog zu Beispiel 21a) lässt sich, ausgehend von 76 Teilen 3-Hydrazinobenzoesäure und 85 Teilen Mucochlorsäure, am Ende der gewünschte Feststoff isolieren.

Ausbeute: 114 Teile (73% d.Th.), Fp.: 134–136 °C
ber. Cl-Gehalt: 22,7%
gef. Cl-Gehalt: 22,6%

b) 5-Chlor-1-(3′-ethoxicarbonylphenyl)-4-fluorpyridazon-6

Wie in Beispiel 21b) beschrieben, werden 85 Teile 4,5-Dichlor-1-(3′-ethoxicarbonylphenyl)-pyridazon-6 durch zweistündige Umsetzung mit 24 Teilen Kaliumfluorid in 150 Teilen trockenem N,N-Dimethylformamid bei 145 °C in das gewünschte Fluorchlorpyridazon umgewandelt.

Ausbeute: 78 Teile (97% d.Th.), Fp.: 117–120 °C
ber. Cl-Gehalt: 12,0% ber. F-Gehalt: 6,4%
gef. Cl-Gehalt: 11,8% gef. F-Gehalt: 6,5%

c) 5-Chlor-1-(3′-chlorocarbonylphenyl)-4-fluorpyridazon-6

Beispiel 21c) entsprechend, werden 78 Teile 5-Chlor-1-(3′-ethoxicarbonylphenyl)-4-fluorpyridazon in das gewünschte Säurechlorid überführt, das als Feststoff anfällt.

Ausbeute: 74 Teile (89% d.Th.), Fp.: 151–154 °C
Analyse:

| | C | H | Cl | F | N | O |
|---|---|---|---|---|---|---|
| ber. | 46,0 | 1,7 | 24,7 | 6,6 | 9,8 | 11,2 |
| gef. | 46,4 | 1,6 | 24,4 | 6,8 | 9,7 | – |

Beispiel 23:
5-Chlor-1-(4'-chlorosulfonylphenyl)-4-fluorpyridazon-6

a) 4,5-Dichlor-1-phenylpyridazon-6

In eine Mischung aus 216 Teilen Phenylhydrazin, 3200 Teilen Wasser und 428 Teilen 38%iger Salzsäure trägt man bei Raumtemperatur 338 Teile Mucochlorsäure ein, erwärmt auf 80 °C, gibt weitere 1500 Teile Wasser zu und lässt 3½ Stdn. lang bei 85 °C rühren. Nach dem Abkühlen auf Raumtemperatur wird das ausgefallene Produkt abgesaugt, mit Wasser gewaschen und getrocknet.

Ausbeute: 478 Teile (99% d.Th.), Fp.: 165–168 °C
ber. Cl-Gehalt: 29,4%
gef. Cl-Gehalt: 29,4%

b) 5-Chlor-4-fluor-1-phenylpyridazon-6

482 Teile 4,5-Dichlor-1-phenylpyridazon-6 werden wie in Beispiel 1b) beschrieben mit 264 Teilen Kaliumfluorid in 650 Teilen trockenem DMF 3 Stunden lang bei 145 °C umgesetzt. Nach wässriger Aufarbeitung isoliert man einen Feststoff.

Ausbeute: 427 Teile (95% d.Th.), Fp.: 144–146 °C
ber. Cl-Gehalt: 15,8% ber. F-Gehalt: 6,8%
gef. Cl-Gehalt: 15,5% gef. F-Gehalt: 8,6%

c) 5-Chlor-1-(4'-chlorosulfonylphenyl)-4-fluorpyridazon-6

200 Teile 5-Chlor-4-fluor-1-phenylpyridazon-6 werden in 1560 Teile Chlorsulfonsäure eingetragen. Man erwärmt das Reaktionsgemsich zunächst auf 80 °C, dann im Verlaufe von 6 Stunden auf 140 °C und lässt 10 Stunden lang bei dieser Temperatur nachrühren. Nach dem Abkühlen auf Raumtemperatur wird das Reaktionsgemisch auf Eis gefällt, rasch abgesaugt und getrocknet. Der erhaltene Feststoff wird mit 1300 Teilen Toluol ausgekocht und von unlöslichen Bestandteilen abfiltriert. Aus dem heissen Filtrat lässt sich durch Zugabe von 1000 Teilen Petrolether das gewünschte Produkt ausfällen, das nach dem Abkühlen auf 0 °C abgesaugt, mit Petrolether gewaschen und dann getrocknet wird.

Ausbeute: 173 Teile (60% d.Th.), Fp.: 144–147 °C
Analyse:

| | C | H | Cl | F | N | O | S |
|---|---|---|---|---|---|---|---|
| ber. | 37,1 | 1,5 | 22,0 | 5,9 | 8,7 | 14,9 | 9,9 |
| gef. | 37,5 | 1,4 | 21,7 | 6,0 | 8,6 | – | 9,8 |

Beispiel 24:
5-Chlor-1-(2'-chlorocarbonylethyl)-4-fluorpyridazon-6

a) 4,5-Dichlor-1-(2'-hydroxicarbonylethyl)pyridazon-6

Zu einem Gemisch aus 400 Teilen Hydrazinhydrat und 200 Teilen Wasser tropft man bei 0 °C 424 Teile Acrylnitril, lässt eine Stunde lang ohne Kühlung nachrühren, gibt 190 Teile 40%ige Salzsäure zu, trägt bei 60–70 °C 1360 Teile Mucochlorsäure portionsweise ein und lässt bei 90 °C eine Stunde lang nachrühren. Nach dem Abkühlen auf Raumtemperatur wird der gebildete Feststoff durch Abdekantieren der überstehenden Flüssigkeit isoliert, mit 1100 Teilen Wasser und dann bei 100–120 °C mit 5300 Teilen 98%iger Schwefelsäure versetzt. Nach einstündigem Nachrühren bei 130 °C gibt man das Reaktionsgemisch auf Eis, saugt den entstandenen Niederschlag ab, wäscht ihn mit Wasser und trocknet ihn im Vakuum.

Ausbeute: 1422 Teile (75% d.YTh.), Fp.: 128–130 °C
ber. Cl-Gehalt: 30,0%
gef. Cl-Gehalt: 30,0%

b) 4,5-Dichlor-1-(2'-ethoxicarbonylethyl)-pyridazon-6

450 Teile 4,5-Dichlor-1-(2'-hydroxicarbonylethyl)-pyridazon-6, 1180 Teile Ethanol und 28 Teile 98%ige Schwefelsäure werden 18 Stunden lang unter Rückfluss gekocht. Anschliessend destilliert man das überschüssige Ethanol im Vakuum ab, wäscht das verbleibende Öl zweimal mit 45 °C warmem Wasser, dann mit 45 °C warmer wässriger Natriumhydrogencarbonatlösung und lässt das Öl durch Zugabe von Eis kristallisieren. Der Feststoff wird abgesaugt, mit Eiswasser gewaschen und getrocknet.

Ausbeute: 472 Teile (94% d.Th.), Fp.: 42–43 °C
ber. Cl-Gehalt: 26,8%
gef. Cl-Gehalt: 26,7%

c) 5-Chlor-1-(2'-ethoxicarbonylethyl)-4-fluorpyridazon-6

319 Teile 4,5-Dichlor-1-(2'-ethoxicarbonylethyl)-pyridazon-6 und 139 Teile Kaliumfluorid werden in 520 Teilen Toluol suspendiert. Anschliessend destilliert man das Toluol ab, gibt 475 Teile trockenes N,N-Dimethylformamid (DMF) zu und lässt 10 Stunden lang bei 140 °C rühren. Die anorganischen Salze werden abfiltriert und mit DMF gewaschen. Aus den vereinigten Filtraten destilliert man zunächst das DMF, dann das Wertprodukt ab.

Ausbeute: 235 Teile (81% d.Th.),
Sdp.:142–143 °C/1,4 mbar
ber. Cl-Gehalt: 14,6% ber. F-Gehalt: 7,8%
gef. Cl-Gehalt: 14,2% gef. F-Gehalt: 8,9%

d) 5-Chlor-4-fluor-1-(2'-hydroxicarbonylethyl)pyridazon-6

200 Teile 5-Chlor-1-(2'-ethoxicarbonylethyl)-4-fluorpyridazon-6 werden in 1600 Teilen 10%iger Salzsäure 48 Stdn. lang bei 60 °C gerührt. Anschliessend engt man die Reaktionslösung im Vakuum bis zur Trockene ein. Es verbleibt ein weisser Feststoff.

Ausbeute: 181 Teile (100%), Fp.: 98–108 °C
ber. Cl-Gehalt: 16,2% ber. F-Gehalt: 8,7%
gef. Cl-Gehalt: 15,9% gef. F-Gehalt: 8,9%

e) 5-Chlor-1-(2'-chlorocarbonylethyl)-4-fluorpyridazon-6

176 Teile 5-Chlor-4-fluor-1-(2'-hydroxicarbonylethyl)-pyridazon-6 werden in 143 Teile Thionylchlorid eingetragen, dann langsam auf 50 °C erwärmt und 8 Stdn. lang bei dieser Temperatur gerührt. Anschliessend entfernt man überschüssiges Thionylchlorid im Vakuum. Es verbleibt ein Öl.

Ausbeute: 190 Teile (100%)

Analyse:

| | C | H | Cl | F | N | O |
|---|---|---|---|---|---|---|
| ber. | 35,1 | 2,1 | 29,7 | 8,0 | 11,7 | 13,4 |
| gef. | 35,6 | 2,0 | 29,4 | 8,1 | 11,6 | – |

Beispiel 25:

5-Chlor-1-(2′-chlorocarbonyl-1′-methylethyl-)-4-fluorpyridazon-6

a) 4,5-Dichlor-1-(2′-hydroxicarbonyl-1′-methylethyl)-pyridazon-6

Wie in Beispiel 24a) beschrieben, setzt man statt 424 Teile Acrylnitril 536 Teile Crotonnitril mit 400 Teilen Hydrazinhydrat und 1360 Teilen Mucochlorsäure um. Nach Aufarbeitung wird ein Feststoff isoliert.

Ausbeute: 682 Teile (68% d.Th.), Fp.: 124–128 °C
ber. Cl-Gehalt: 28,3%
gef. Cl-Gehalt: 28,4%

b) 4,5-Dichlor-1-(2′-ethoxicarbonyl-1′-methylethyl)-pyridazon-6

Analog zu Beispiel 24b) lassen sich 477 Teile 4,5-Dichlor-1-(2′-hydroxicarbonyl-1′-methylethyl)-pyridazon-6 in Ethanol verestern. Es verbleibt ein Feststoff.

Ausbeute: 440 Teile (83% d.Th.), Fp.: 40–45 °C
ber. Cl-Gehalt: 25,4%
gef. Cl-Gehalt: 25,2%

c) 5-Chlor-1-(2′-ethoxicarbonyl-1′-methylethyl-4-fluor-pyridazon-6

Analog Beispiel 24c) werden 336 Teile 4,5-Dichlor-1-(2′-ethoxicarbonyl-1′-methylethyl)-pyridazon-6 mit 139 Teilen Kaliumfluorid umgesetzt. Das Produkt fällt als Öl an.

Ausbeute: 272 Teile (86% d.Th.), Sdp.: 128–130 °C/0,7 mbar
ber. Cl-Gehalt: 13,5% ber. F-Gehalt: 7,2%
gef. Cl-Gehalt: 13,5% gef. F-Gehalt: 7,2%

d) 5-Chlor-4-fluor-1-(2′-hydroxicarbonyl-1′-methylethyl)-pyridazon-6

Wie in Beispiel 24d) beschrieben, werden 211 Teile 5-Chlor-1-(2′-ethoxicarbonyl-1′-methylethyl)-4-fluorpyridazon-6 in 10%iger Salzsäure hydrolysiert. Man erhält einen weissen Feststoff.

Ausbeute: 188 Teile (100%), Fp.: 67–69 °C
ber. Cl-Gehalt: 15,1% ber. F-Gehalt: 8,1%
gef. Cl-Gehalt: 14,9% gef. F-Gehalt: 7,9%

e) 5-Chlor-1-(2′-chlorocarbonyl-1′-methylethyl)-4-fluorpyridazon-6

Analog zu Beispiel 24e) lassen sich 185 Teile 5-Chlor-4-fluor-1-(2′-hydroxycarbonyl-1′-methylethyl)-pyridazon-6 mittels Thionylchlorid in das als Öl anfallende gewünschte Säurechlorid überführen.

Ausbeute: 199 Teile (100%)

Analyse:

| | C | H | Cl | F | N | O |
|---|---|---|---|---|---|---|
| ber. | 37,9 | 2,8 | 28,1 | 7,5 | 11,1 | 12,6 |
| gef. | 38,3 | 2,6 | 28,0 | 7,4 | 11,0 | – |

Beispiel 26:

5-Chlor-1-(2′-chlorocarbonylethyl)-4-fluor-3-nitropyridazon-6

a) 5-Chlor-4-fluor-1-(2′-hydroxicarbonylethyl)-3-nitropyridazon-6

Zu einer Lösung von 373 Teilen 5-Chlor-4-fluor-1-(2′-hydroxicarbonylethyl)-pyridazon-6 (Herstellung siehe Beispiel 24d) in 558 Teilen 98%iger Schwefelsäure und 1523 g 24%igem Oleum gibt man portionsweise unter Eiskühlung 416 Teile Kaliumnitrat, heizt dann langsam auf 60 °C und lässt 2 Stunden lang bei dieser Temperatur nachrühren. Nach dem Abkühlen wird das Reaktionsgemisch auf Eis gegeben, der entstandene Niederschlag abgesaugt, mit Wasser gewaschen, getrocknet und aus Toluol umkristallisiert.

Ausbeute: 194 Teile (43% d.Th.), Fp.: 157–161 °C
ber. Cl-Gehalt: 13,4% ber. F-Gehalt: 7,2%
gef. Cl-Gehalt: 13,3% gef. F-Gehalt: 7,1%

b) 5-Chlor-1-(2′-chlorocarbonylethyl)-4-fluor-3-nitropyridazon-6

Analog zu Beispiel 24e) werden 190 Teile 5-Chlor-4-fluor-1-(2′-hydroxicarbonylethyl)-3-nitropyridazon-6 durch Umsetzung mit Thionylchlorid in das gewünschte Säurechlorid umgewandelt, das als Feststoff anfällt.

Ausbeute: 203 Teile (100% d.Th.), Fp.: 104–108 °C

Analyse:

| | C | H | Cl | F | N | O |
|---|---|---|---|---|---|---|
| ber. | 29,6 | 1,4 | 25,0 | 6,7 | 14,8 | 22,5 |
| gef. | 30,0 | 1,4 | 25,0 | 6,5 | 14,7 | – |

Beispiel 27:

1-(1′,2′-Bischlorocarbonylethyl)-5-chlor-4-fluorpyridazon-6

a) 1-(1′,2′-Bishydroxicarbonylethyl)-4,5-dichlorpyridazon-6

In ein Gemisch aus 215 Teilen Hydrazinhydrat und 108 Teilen Wasser trägt man bei 20–30 °C portionsweise 335 Teile Fumarsäuredinitril ein, lässt eine Stunde lang bei 30 °C nachrühren, gibt 103 Teile 40%ige Salzsäure zu, trägt bei 50 °C 731 Teile Mucochlorsäure portionsweise ein und lässt bei 90 °C zwei Stunden lang nachrühren. Nach dem Abkühlen auf Raumtemperatur wird das Rohprodukt durch Abdekantieren der überstehenden Flüssigkeit isoliert, mit 3700 Teilen Wasser sowie mit 5700 Teilen 98%iger Schwefelsäure versetzt. Nach fünfzehnstündigem Nachrühren bei 130 °C gibt man das Reaktionsgemisch auf Eis, saugt den sich bildenden Niederschlag ab, wäscht ihn mit Wasser und trocknet ihn im Vakuum.

Ausbeute: 568 Teile (47% d.Th.), Fp.: 179–181 °C
ber. Cl-Gehalt: 25,3%
gef. Cl-Gehalt: 25,0%

b) 1-(1′,2′-Bisethoxicarbonylethyl)-4,5-dichlorpyridazon-6

540 Teile 1-(1′,2′-Bishydroxicarbonylethyl)-4,5-dichlorpyridazon-6, 3160 Teile Ethanol und 110 Teile 98%ige Schwefelsäure werden 36 Stunden lang unter Rückfluss gekocht. Nach Aufarbeitung wie in Beispiel 24b) isoliert man ein Öl.

Ausbeute: 480 Teile (74% d.Th.)
ber. Cl-Gehalt: 21,1%
gef. Cl-Gehalt: 21,2%

c) 1-(1′,2′-Bisethoxicarbonylethyl)-5-chlor-4-fluorpyridazon-6

472 Teile 1-(1′,2′-Bisethoxicarbonylethyl)-4,5-dichlorpyridazon-6 werden analog zu Beispiel 24c) mit 244 Teilen Kaliumfluorid in DMF bei 140 °C 12 Stunden lang umgesetzt. Nach Aufarbeitung isoliert man ein Öl.

Ausbeute: 401 Teile (89% d.Th.), Sdp. 166–169 °C/0,6 mbar

ber. Cl-Gehalt: 11,1% ber. F-Gehalt: 5,9%

gef. Cl-Gehalt: 10,9% gef. F-Gehalt: 6,1%

d) 1-(1′,2′-Bishydroxicarbonylethyl)-5-chlor-4-fluorpyridazon-6

werden in 2000 Teilen 10%iger Schwefelsäure 15 Stunden lang bei 60 °C gerührt. Nach dem Abkühlen auf Raumtemperatur extrahiert man das Reaktionsgemisch dreimal mit Essigester, trocknet die vereinigten Extrakte über Natriumsulfat, filtriert vom Trockenmittel ab und entfernt aus dem Filtrat das Lösungsmittel. Es verbleibt ein Öl.

Ausbeute: 241 Teile (91% d.Th.)

ber. Cl-Gehalt: 13,4% ber. F-Gehalt: 7,2%

gef. Cl-Gehalt: 13,3% gef. F-Gehalt: 7,0%

e) 1-(1′,2′-Bischlorocarbonylethyl)-5-chlor-4-fluorpyridazon-6

240 Teile 1-(1′,2′-Bishydroxycarbonylethyl)-5-chlor-4-fluropyridazon-6 werden Beispiel 24e) entsprechend mit 400 Teilen Thionylchlorid umgesetzt. Nach dem Abdestillieren überschüssigen Thionylchlorids verbleibt ein Öl.

Ausbeute: 273 Teile (100% d.Th.)

Analyse:

| | C | H | Cl | F | N | O |
|---|---|---|---|---|---|---|
| ber. | 31,9 | 1,3 | 35,3 | 6,3 | 9,3 | 15,9 |
| gef. | 32,3 | 1,4 | 35,0 | 6,2 | 9,2 | – |

Beispiel 28:

5-Chlor-1-chlorocarbonylmethyl-4-fluorpyridazon-6

a) 4,5-Dichlor-1-ethoxicarbonylmethylpyridazon-6

145 Teile Hydrazinoessigsäureethylesterhydrochlorid und 157 Teile Mucochlorsäure werden in 1500 Teilen Wasser 15 Minuten lang unter Rückfluss gekocht. Nach dem Abkühlen saugt man das ausgefallene Produkt ab, wäscht es mit Wasser, trocknet es, trägt es in eine Mischung aus 500 Teilen Ethanol und 9 Teilen 98%iger Schwefelsäure ein und lässt 7 Stunden lang unter Rückfluss kochen. Nach dem weitgehenden Entfernen des Ethanols im Vakuum wird der Rückstand mit Eiswasser verrührt, abgesaugt, mit Wasser gewaschen und getrocknet.

Ausbeute: 210 Teile (90% d.Th.), Fp.: 95–96 °C

ber. Cl-Gehalt: 28,3%

gef. Cl-Gehalt: 28,2%

b) 5-Chlor-1-ethoxicarbonylmethyl-4-fluorpyridazon-6

201 Teile 4,5-Dichlor-1-ethoxicarbonylmethylpyridazon-6 werden wie in Beispiel 24c) beschrieben mit Kaliumfluorid in DMF umgesetzt. Das Produkt fällt nach Destillation als Öl an.

Ausbeute: 118 Teile (63% d.Th.), Sdp. 148–150 °C/3 mbar

ber. Cl-Gehalt: 15,1% ber. F-Gehalt: 8,1%

gef. Cl-Gehalt: 15,4% gef. F-Gehalt: 8,1%

c) 5-Chlor-4-fluor-1-hydroxicarbonylmethylpyridazon-6

117 Teile 5-Chlor-1-ethoxicarbonylmethyl-4-fluorpyridazon-6 werde wie in Beispiel 24d) beschrieben durch 10%ige Salzsäure hydrolysiert. Es entsteht ein Feststoff.

Ausbeute: 103 Teile (100% d.Th.), Fp.: 149–153 °C (aus Toluol)

ber. Cl-Gehalt: 17,2% ber. F-Gehalt: 9,2%

gef. Cl-Gehalt: 17,3% gef. F-Gehalt: 9,1%

d) 5-Chlor-1-chlorocarbonylmethyl-4-fluorpyridazon-6

100 Teile 5-Chlor-fluor-1-hydroxicarbonylmethylpyridazon-6 werden Beispiel 24e) entsprechend mittels Thionylchlorid in das als Feststoff anfallende Säurechlorid überführt.

Ausbeute: 109 Teile (100% d.Th.), Fp.: 164–166 °C (aus Toluol)

Analyse:

| | C | H | Cl | F | N | O |
|---|---|---|---|---|---|---|
| ber. | 32,0 | 1,3 | 31,6 | 8,4 | 12,5 | 14,2 |
| gef. | 32,3 | 1,2 | 31,8 | 8,2 | 12,4 | – |

Analog zu den Beispielen 21–28 konnten folgende 4-Fluorpyridaz-6-one synthetisiert werden:

| Beispiel Nr. | |
|---|---|
| 29 | 5-Chlor-1-(2′-chlorocarbonylpropyl)-4-fluorpyridazon-6 |
| 30 | 5-Brom-1-(4′-chlorocarbonylphenyl)-4-fluorpyridazon-6 |
| 31 | 5-Brom-1-(3′-chlorocarbonylphenyl)-4-fluorpyridazon-6 |
| 32 | 5-Brom-1-(4′-chlorosulfonylphenyl)-4-fluorpyridazon-6 |
| 33 | 5-Brom-1-(2′-chlorocarbonylethyl)-4-fluorpyridazon-6 |
| 34 | 5-Brom-1-(2′-chlorocarbonylethyl)-4-fluor-3-nitropyridazon-6 |
| 35 | 5-Brom-1-chlorocarbonylmethyl-4-fluorpyridazon-6 |

**Patentansprüche**

1. Substituierte 4-Fluorpyridaz-6-one der allgemeinen Formel

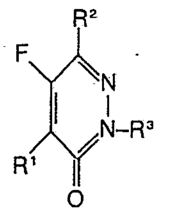

(I)

in der

$R^1$ Fluor, Chlor oder Brom,

$R^2$ Wasserstoff oder Nitro und

$R^3$ durch Chlorocarbonyl, Chlorosulfonyl, Amino, Isocyanato oder Isothiocyanato substituiertes Alkyl, Phenyl oder Naphthyl oder ein ebenso substituierter Heterocyclus sind, wobei das Alkyl noch durch Fluor, Chlor, Brom, $C_1$- bis $C_4$-Alkoxy oder Hydroxycarbonyl und das Phenyl, Naphthyl und der Heterocyclus darüber hinaus durch Hydroxy, Trifluormethyl oder Hydroxylsulfonyl weiter substituiert sein können.

2. Verbindungen gemäss Anspruch 1, bei denen $R^1$ Chlor und $R^2$ Wasserstoff sind und $R^3$ die angegebene Bedeutung hat.

3. Verbindungen gemäss Anspruch 1, bei denen $R^3$ Chlorocarbonylethyl ist.

**Claims**

1. A substituted 4-fluoropyridaz-6-one of the general formula

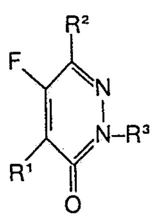

(I)

where

$R^1$ is fluorine, chlorine or bromine,

$R^2$ is hydrogen or nitro, and

$R^3$ is alkyl, phenyl or naphthyl substituted by chlorocarbonyl, chlorosulfonyl, amino, isocyanato or isothiocyanato, or is a similarly substituted heterocyclic radical, it being possible for the alkyl to be further substituted by fluorine, chlorine, bromine, $C_1$–$C_4$-alkoxy or hydroxycarbonyl, and for the phenyl, naphthyl and heterocyclic radicals to be further substituted by hydroxyl, trifluoromethyl or hydroxysulfonyl.

2. A compound as claimed in claim 1, wherein $R^1$ is chlorine, $R^2$ is hydrogen and $R^3$ has the specified meanings.

3. A compound as claimed in claim 1, wherein $R^3$ is chlorocarbonylethyl.

**Revendications**

1. 4-fluoropyridazones-6 substituées de formule générale

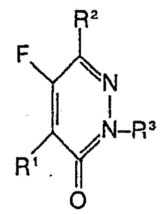

(I)

dans laquelle

$R^1$ est un atome de fluor, de chlore ou de brome,

$R^2$ est un atome d'hydrogène ou un groupement nitro, et

$R^3$ est un radical alkyle, phényle ou naphthyle substitué par un groupement chlorocarbonyle, chlorosulfonyle, amino, isocyanato ou isothiocyanato ou est un hétérocycle substitué de même, le radical alkyle pouvant être encore substitué par un atome de fluor, de chlore, de brome, par un groupement alcoxy en $C_1$ à $C_4$ ou hydroxycarbonyle et le radical phényle, naphthyle et l'hétérocycle pouvant être en outre substitués par un groupement hydroxy, trifluorométhyle ou hydroxysulfonyle.

2. Composés selon la revendication 1, dans lesquels $R^1$ est un atome de chlore, $R^2$ est un atome d'hydrogène et $R^3$ a la signification donnée dans cette revendication.

3. Composés selon la revendication 1, dans lesquels $R^3$ est un groupement chlorocarbonyléthyle.